# EUROPÄISCHE PATENTSCHRIFT

(11) **EP 2 661 220 B1**
(45) Veröffentlichungstag und Bekanntmachung des Hinweises auf die Patenterteilung: **17.09.2014**
(21) Anmeldenummer: 11808558.8
(22) Anmeldetag: 15.12.2011
(51) Int. Cl.: A61B 5/0408, A61B 5/0478

(54) **MEDIZINISCHE ELEKTRODE MIT GEDRUCKTER, ABGESCHIRMTER ZULEITUNG**
MEDICAL ELECTRODE WITH PRINTED SHIELDED FEED LINE
ÉLECTRODE MÉDICALE COMPRENANT UNE CONDUITE D'ALIMENTATION BLINDÉE IMPRIMÉE

(30) Priorität: 03.01.2011 AT 42011
(43) Veröffentlichungstag der Anmeldung: 13.11.2013
(73) Patentinhaber: Leonh. Lang, 6020 Innsbruck (AT)
(72) Erfinder: LANG, Burrhus, A-6020 Innsbruck (AT)
(74) Vertreter: Torggler, Paul Norbert
(86) Internationale Anmeldenummer: PCT/AT2011/000496
(87) Internationale Veröffentlichungsnummer: WO 2012/092632

(56) Entgegenhaltungen:
- EP-A2- 1 752 093
- US-A- 4 442 315
- US-A1- 2007 299 471
- US-A1- 2010 139 943

## Beschreibung

Die Erfindung betrifft eine medizinische Elektrode mit einem Elektrodenkopf und einer elektrischen Zuleitung zum Elektrodenkopf, wobei die Zuleitung eine elektrisch leitende Abschirmschicht, ein dielektrisches Element und einen elektrischen Leiter aufweist, wobei die Zuleitung ein längliches Trägerelement aufweist, auf das die elektrisch leitende Abschirmschicht zumindest teilweise aufgedruckt ist, sowie ein Verfahren zur Herstellung einer solchen medizinischen Elektrode.

Medizinische Elektroden zum Ein- bzw. Ausleiten elektrischer Ströme in den bzw. vom menschlichen oder tierischen Körper sind schon seit geraumer Zeit bekannt. Es gibt auf diesem Gebiet auch die unterschiedlichsten Herstellungsmethoden, wobei es in den letzten Jahren immer wichtiger wurde, möglichst kleine und leichte Elektroden herzustellen. Auf dem Sektor der Diagnoseelektroden gibt es auch zunehmend das Bestreben, eine möglichst von elektromagnetischer Strahlung und anderen Störquellen unbeeinflusste Auswertung zu ermöglichen. Dazu werden neben entsprechenden Elektrodendesigns vor allem geschirmte Kabel verwendet. Diese bewahren das abgenommen Signal vor Störungen durch elektromagnetische Strahlung und schützen gegebenenfalls auch den Patienten.

Um vor allem den Elektrodenkopf - mit dem die medizinische Elektrode am Körper angebracht wird - möglichst einfach und dünn herzustellen, ist aus dem Stand der Technik die Möglichkeit bekannt, zumindest einzelne Schichten in einem Druckverfahren aufzubringen. Beispielsweise zeigt die US 2010/0030167 A1 eine Elektrode mit elektrisch leitenden Ringen und eine weitere Abschirmungsschicht als zweite elektrisch leitende Schicht. Diese zweite elektrisch leitende Schicht kann auf die Elektrode gedruckt werden.

Gemäß der WO 2009/007877 A2 ist hauptsächlich ein Elektrodenkopf beschrieben, bei dem eine leitende Tinte auf die Oberfläche einer Filmschicht aufgebracht wird. Es kann auch vorgesehen sein, dass eine dielektrische Schicht über einen Grundleiter gedruckt ist.

Weiters ist es aus der DE 699 23 680 T2 bekannt, dass bei einer Elektrode über einem Schaltkreis aus leitender Tinte eine Sperrschicht (im chemischen Sinn) aus einer Silberlegierungstinte aufgedruckt ist.

Zudem zeigt die DE 40 91 800 C2 eine Biosignalelektrode mit einer zweischichtig aufgebauten, elektrisch leitfähigen, aufgedruckten Schicht.

Im technischen Gebiet der Zuleitung zum Elektrodenkopf sind im Querschnitt runde, geschirmte und ungeschirmte Kabel bekannt. Für eine den Elektrodenkopf in die Schirmung integrierende Verbindung müssen entweder die Anschlüsse und dazugehörigen Stecker relativ aufwändig gestaltet werden, wie in der EP 1 569 551 B1 gezeigt wird, oder der Stecker zusätzlich als den Elektrodenkopf überlappende Schirmung ausgeführt werden, was diesen Bereich schwer und unflexibel werden lässt und im Übrigen einer in die Elektrode integrierten Schirmung qualitativ unterlegen ist.

Nachteilig ist somit grundsätzlich, dass die Herstellung einer medizinischen Elektrode mit Elektrodenkopf und integrierter, ungeschirmter und runder Kabelzuleitung immer sehr umständlich, aufwändig und üblicherweise in mehreren komplett voneinander getrennten Schritten, die zum Teil auf manueller Montage basieren, durchgeführt wird, weshalb die Herstellungsprozesse solcher medizinischer Elektroden sehr ineffizient sind.

Diese Schwierigkeiten - und damit die Kosten - potenzieren sich geradezu, wenn nun eine Elektrode mit geschirmtem Elektrodenkopf und integrierter geschirmter Kabelzuleitung gefertigt werden soll, weswegen derartige Einmalprodukte am Markt wohl so gut wie nicht zu finden sind.

Weiters ist auf dem Gebiet der Zuleitung zum Elektrodenkopf einer medizinischen Elektrode aus der US 4,353,372 A bekannt, dass sowohl der elektrische Leiter auf der Zuleitung als auch der Leiter im Bereich des Verbindungssteckers als leitende Schicht aufgedruckt sind.

Aus der US 4,442,315 A und der US 2007/0299471 A1 sind jeweils medizinische Elektroden mit Elektrodenkopf und Zuleitung bekannt, bei denen im Bereich der Zuleitung die Abschirmschichten bzw. Isolierschicht aufgedruckt werden können. Bei diesen aufgedruckten Abschirmschichten ist allerdings keine komplette Abschirmung der elektrischen Leiter gegeben, sodass elektromagnetische Strahlung oder andere Störquellen die durch den elektrischen Leiter in der Zuleitung geführten Signale beeinflussen können.

Das Dokument US 2007/0299471 A1 beschreibt eine medizinische Elektrode mit einem Elektrodenkopf und einer elektrischen Zuleitung, wobei die Zuleitung eine elektrisch leitende Abschirmschicht, ein dielektrisches Element und einen elektrischen Leiter aufweist. Die elektrisch leitende Abschirmschicht ist zumindest teilweise auf ein längliches Trägerelement aufgedruckt, und umfasst eine obere und eine untere Abschirmschicht.

Die Aufgabe der vorliegenden Erfindung besteht daher darin, eine gegenüber dem Stand der Technik verbesserte medizinische Elektrode anzugeben. Insbesondere sollen die bisher bekannten (geschirmten) Zuleitungskabel besser handhabbar werden. Zudem sollen die elektrischen Verbindungen zwischen Elektrodenkopf und einer medizinischen Auswerte- bzw. Einleitungseinheit weniger aufwändig gestaltet und effizienter hergestellt werden können. Es sollen auch die durch den elektrischen Leiter in der Zuleitung übermittelten Signale möglichst unbeeinflusst von Störquellen sein.

Dies wird für eine medizinische Elektrode mit den Merkmalen des Oberbegriffes von Anspruch 1 dadurch gelöst, dass die zumindest teilweise gedruckte Abschirmschicht das dielektrische Element und den elektrischen Leiter quer zur Längsachse der Zuleitung allseitig umgibt, wobei die zumindest teilweise gedruckte Abschirmschicht eine obere und eine untere elektrische Abschirmschicht umfasst, wobei die obere und die untere elektrische Abschirmschicht zumindest bereichsweise stoffschlüssig verbunden sind. Dadurch, dass zumindest ein Teil der Abschirmung gedruckt ist, kann eine wesentlich einfachere Herstellung und eine genauer auf die Bedürfnisse abgestimmte Produktion der medizinischen Elektrode erreicht werden.

Um eine besonders effiziente und schnelle Herstellung zu erreichen, kann besonders bevorzugt vorgesehen sein, dass auch das dielektrische Element und/oder der elektrische Leiter gedruckt sind.

Eine Ausführungsvariante kann vorsehen, dass das Trägerelement als metallisierte Folie ausgeführt ist, sodass das Trägerelement bereits bereichsweise als Schirmung wirkt. Auf diese metallisierte Folie wird der elektrische Leiter samt Dielektrikum aufgebracht, bevorzugt aufgedruckt, und anschließend wird erst der gedruckte Teil der Abschirmung aufgedruckt und verbindet sich mit der metallisierten Folie derart, dass der elektrische Leiter samt Dielektrikum quer zur Längsrichtung der Zuleitung allseitig von der (zweiteiligen) Abschirmung umgeben ist.

Gemäß einer besonders bevorzugten Ausführungsform der vorliegenden Erfindung kann jedoch vorgesehen sein, dass die elektrisch leitende Abschirmschicht, das dielektrische Element und der elektrische Leiter gedruckt sind, wobei bevorzugt die Abschirmschicht zumindest teilweise direkt auf das Trägerelement und das dielektrische Element und der elektrische Leiter indirekt auf das Trägerelement aufgedruckt sind. Das heißt, dass das dielektrische Element und der elektrische Leiter den eigentlichen Träger nicht direkt berühren, sondern schichtweise aufeinander gedruckt sind.

Eine Zuleitung in Form eines zumindest teilweise gedruckten "Kabels" kann verbessert werden, indem die Zuleitung ein oberes und unteres dielektrisches Element aufweist, das den elektrischen Leiter quer zur Längsachse der Zuleitung umgibt.

Die Vorteile des Druckens zumindest einzelner Schichten können nicht nur für die Zuleitung verwendet werden, sondern es ist bevorzugt auch vorgesehen, dass der Elektrodenkopf eine elektrische leitende Signalabgabe- bzw. -aufnahmeschicht, die mit dem elektrischen Leiter der Zuleitung verbunden ist, ein dielektrisches Element, das mit dem dielektrischen Element der Zuleitung verbunden ist und eine Abschirmschicht, die mit der Abschirmschicht der Zuleitung verbunden ist, aufweist, wobei zumindest die elektrisch leitende Signalabgabe- bzw. -aufnahmeschicht des Elektrodenkopfs, die dielektrische Schicht des Elektrodenkopfs und/oder die Abschirmschicht des Elektrodenkopfs gedruckt ist. Es können auch zumindest zwei oder alle drei dieser Bestandteile des Elektrodenkopfes gedruckt werden.

Für eine besonders einfache Herstellung kann diesbezüglich vorgesehen sein, dass auch der Elektrodenkopf ein elektrisch nichtleitendes Trägerelement aufweist, wobei die Abschirmschicht des Elektrodenkopfs direkt auf dem Trägerelement des Elektrodenkopfs aufgedruckt ist.

Um den Komfort für den Patienten zu erhöhen, einen besseren Schutz vor Umwelteinflüssen zu erreichen und eine längere Haltbarkeit zu garantieren, kann bevorzugt vorgesehen sein, dass eine, vorzugsweise aus Kunststoff bestehende, Schutzschicht die elektrische Zuleitung zumindest teilweise umgibt. Bevorzugt umschließt diese Schutzschicht bzw. Hüllschicht die anderen Teile der Zuleitung quer zur Längsachse der Zuleitung vollständig.

Für ein sicheres und einfaches Abgreifen bzw. Einleiten von elektrischem Strom kann bevorzugt vorgesehen sein, dass an das elektrodenkopfabgewandte Ende der Zuleitung ein Verbindungsstecker anbringbar bzw. angebracht ist, über den die medizinische Elektrode mit einer medizinischen Auswerte- und/oder Signaleinleitungseinheit verbindbar ist. Durch die Ausführung der Zuleitung mit teilweise gedruckten Elementen ist eine besonders einfache Anschließbarkeit an einen Verbindungsstecker möglich.

Grundsätzlich ist es zwar möglich, dass nur einzelne Bereiche bzw. einzelne Teile der Zuleitung und des Elektrodenkopfes gedruckt sind, jedoch ist besonders bevorzugt vorgesehen, dass die gesamte Abschirmschicht, das gesamte dielektrische Element und/oder der gesamte elektrische Leiter zwischen Elektrodenkopf und Verbindungsstecker gedruckt ist/sind. Das heißt, Abschirmung, Dielektrikum und elektrischer Leiter sowohl des Elektrodenkopfes als auch der Zuleitung können in einem schnellen, wenige Schritte umfassenden Druckverfahren hergestellt werden.

Schutz wird daher auch begehrt für ein Verfahren mit den Merkmalen des Anspruches 12. Diese Schritte werden dabei bevorzugt in der im Anspruch angegebenen Reihenfolge ausgeführt, wobei für das Herstellen einer rundum sicheren Zuleitung das Drucken eines oberen, dielektrischen Elements unter Einschluss des elektrischen Leiters auf das untere, dielektrische Element zumindest im Bereich der Zuleitung, wobei mit dem Drucken das obere und untere dielektrische Element zumindest bereichsweise stoffschlüssig verbunden werden, und das Drucken einer oberen, dielektrischen Abschirmschicht unter Einschluss des oberen und unteren dielektrischen Elements auf die untere Abschirmschicht zumindest im Bereich der Zuleitung, wobei mit dem Drucken die obere und untere Abschirmschicht zumindest bereichsweise stoffschlüssig verbunden werden, vorgesehen sind.

Besonders gut geeignet ist ein solches Herstellungsverfahren zum Herstellen einer Mehrfachelektrode mit zumindest zwei separaten Signalabgabe- bzw. - aufnahmeschichten, wobei auch zumindest zwei separate Abschirmungsschichten, zumindest zwei separate dielektrische Elemente und zumindest zwei separate elektrische Leiter auf ein gemeinsames Trägerelement gedruckt werden. Dieses Trägerelement kann natürlich nach dem Bedrucken auch teilweise zugeschnitten bzw. eingeschnitten werden, sodass eine größere Erreichbarkeit mit der Zuleitung gegeben ist.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen:
- Fig. 1: eine schematische Ansicht einer medizinischen Elektrode mit den Schnitten
A-A und B-B,
- Fig. 2: einen Querschnitt durch ein Zuleitungskabel nach dem Stand der Technik,
- Fig. 3: einen Querschnitt durch ein Ausführungsbeispiel einer erfindungsgemäßen Zuleitung,
- Fig. 4: einen Querschnitt durch eine alternative Ausführung einer Zuleitung,
- Fig. 5: schematisch eine Druckvorrichtung,
- Fig. 6: eine Draufsicht auf ein bedrucktes Trägerelement und
- Fig. 7 und 8: schematische Ansichten von Mehrfachelektroden.

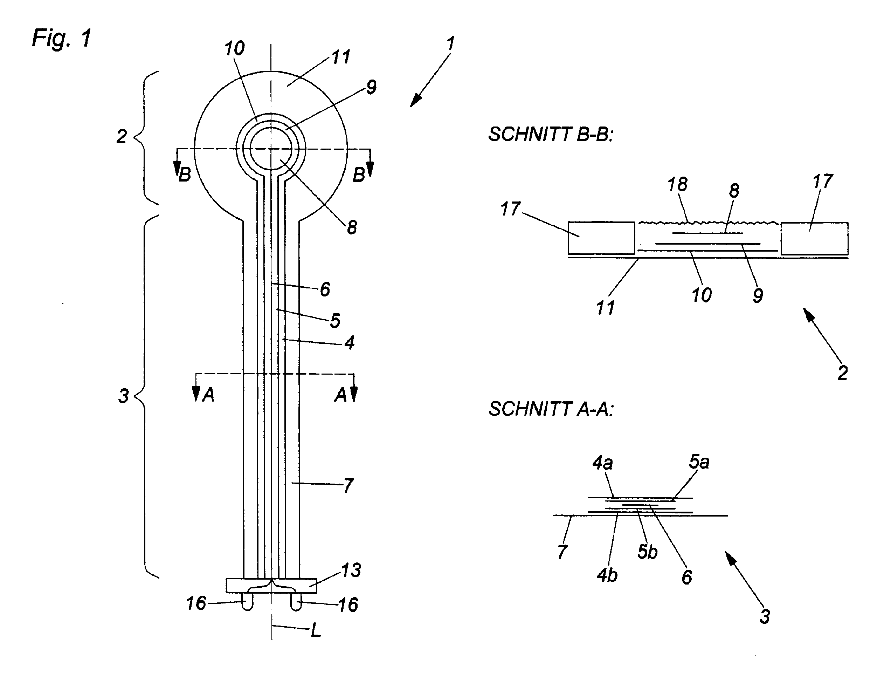

Fig. 1 zeigt eine medizinische Elektrode 1, welche einen Elektrodenkopf 2, eine Zuleitung 3 und einen Verbindungsstecker 13 (mit in diesem Fall vorstehenden Anschlüssen 16) aufweist. Gemäß Fig. 1 ist das Trägerelement 7 der Zuleitung 3 und das Trägerelement 11 des Elektrodenkopfes 2 einstückig ausgebildet, worauf als nächste Schicht die Abschirmschicht 4 bzw. 10 aufgedruckt ist. Anschließend wird darauf das dielektrische Element 5 bzw. 9 und darauf der elektrische Leiter 6 bzw. die Signalabgabe- bzw. -aufnahmeschicht 8 gedruckt.

Im Schnitt A-A ist ein schematischer Querschnitt im Bereich der Zuleitung 3 gezeigt. wobei auf das Trägerelement 7 zuerst die untere Abschirmschicht 4b und das untere dielektrische Element 5b aufgedruckt ist. Oberhalb des dann aufgebrachten elektrischen Leiters 6 ist das obere dielektrische Element 5a und die obere Abschirmschicht 4a aufgedruckt.

Im Schnitt B-B sind schematisch im Querschnitt die wesentlichen Bestandteile des Elektrodenkopfes 2 ersichtlich, wobei auf das Trägerelement 11 die Abschirmschicht 10, das dielektrische Element 9 und die Signalabgabe- bzw. -aufnahmeschicht 8 aufgedruckt ist. Im seitlichen Bereich werden diese aufgedruckten Schichten beispielsweise durch einen Schaumstoff 17 begrenzt. Für eine bessere Stromab- bzw. -einleitung kann, wie an sich bekannt, ein Gel 18 auf den Elektrodenkopf 2 aufgebracht werden.

Grundsätzlich sei darauf hingewiesen, dass in den Zeichnungen die Grenze zwischen Elektrodenkopf 2 und Zuleitung 3 nur angedeutet ist. Mit Worten beschrieben liegt diese Grenze da, wo der elektrische Leiter 6 nicht mehr vom oberen dielektrischen Element 5a oder von der oberen Abschirmschicht 4a bedeckt ist und somit ohne diesen Schutz als Signalabgabe- bzw. -aufnahmeschicht 8 fungieren kann.

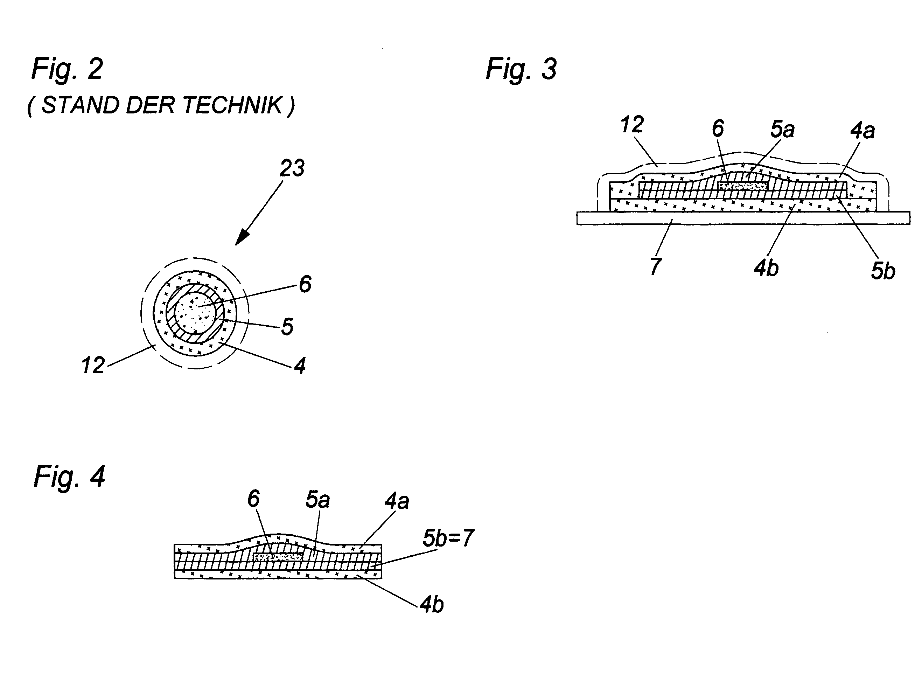

Fig. 2 zeigt grundsätzlich einen Schnitt durch ein bei medizinischen Elektroden bereits eingesetztes rundes, abgeschirmtes Kabel 23 mit einem elektrischen Leiter 6, einem Dielektrikum 5, einer Abschirmung 4 und gegebenenfalls einer Schutzhülle 12.

Da neben der Handhabung vor allem die Herstellung eines solchen bekannten abgeschirmten Kabels 23 relativ schlecht und nachteilig ist, ist erfindungsgemäß vorgesehen, dass die Zuleitung 3 ein längliches Trägerelement 7 aufweist, auf das zumindest ein Teil der elektrisch leitenden Abschirmschicht 4 aufgedruckt ist. Eine besonders bevorzugte Ausführungsform diesbezüglich ist aus der Fig. 3 ersichtlich. Dabei ist auf das Trägerelement 7 die untere Abschirmschicht 4b und darüber das dielektrische Element 5b aufgedruckt. Nach dem Aufdrucken des elektrischen Leiters 6 wird dieser vom oberen dielektrischen Element 5a überdruckt, wodurch der elektrische Leiter 6 quer zur Längsachse L komplett vom dielektrischen Element 5 umschlossen ist, da sich die Elemente 5a und 5b zumindest bereichsweise stoffschlüssig verbinden. Dasselbe gilt für die Abschirmschichten 4a und 4b, die ihrerseits wiederum das dielektrische Element 5 quer zur Längsachse L allseitig umschließen. Weiters kann gemäß der Strichlierung eine Schutzhülle 12 beispielsweise mittels eines Lacksprays aufgebracht werden.

Fig. 4 zeigt eine alternative Ausführungsform, bei der kein separates Trägerelement 7 vorgesehen ist, sondern vielmehr das untere dielektrische Element 5b als Trägerelement 7 ausgebildet ist. Die Abschirmschichten 4a und 4b sind in Fig. 4 in einer nicht erfindungsgemäßen Ausführungsform dargestellt.

Grundsätzlich soll bei allen Varianten nicht ausgeschlossen werden, dass zumindest einzelne Schichten (5a, 5b und 6) nicht im Druckverfahren aufgebracht werden, sondern z.B. auch als einzelne Folien beim Herstellungsprozess zwischen den anderen Schichten eingebracht werden (z.B. in Form einer Klebefolie oder in Form eines Drahtes).

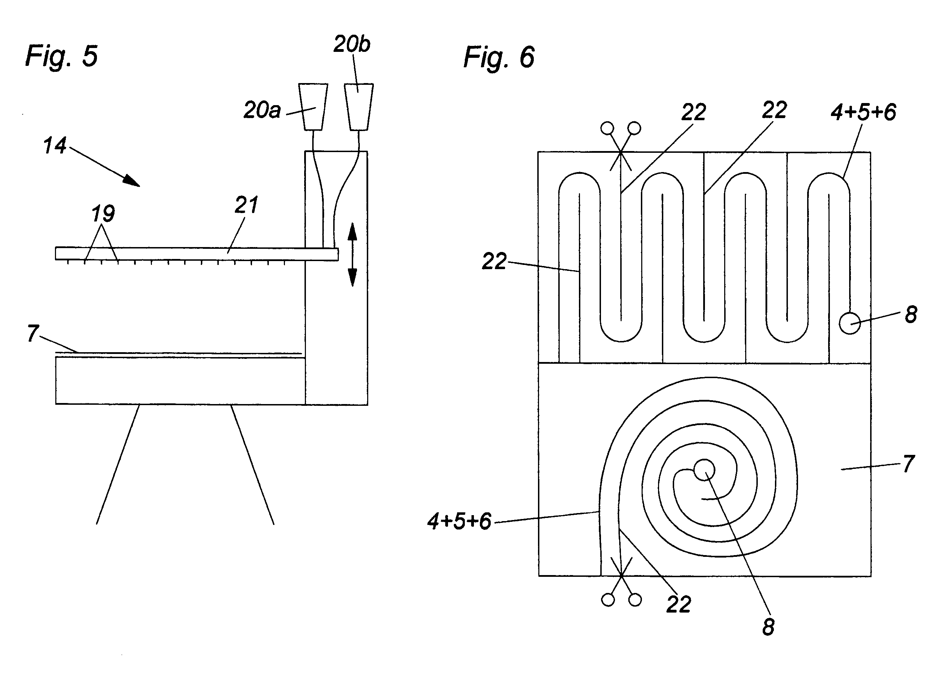

In Fig. 5 ist schematisch eine Druckvorrichtung 14 dargestellt, mit der zumindest einzelne Schichten der medizinischen Elektrode 1 gedruckt werden können. Grundsätzlich soll nicht ausgeschlossen werden, dass das Drucken in einem Inlineverfahren oder auch durch Tintenstrahldrucken erfolgt. Bevorzugt ist allerdings das Siebdruckverfahren vorgesehen, bei dem ein bis zu mehrere Quadratmeter großer Bogen, bevorzugt in Form eines Kunststoffträgerelementes 7, in die Druckvorrichtung 14 eingelegt wird. Anschließend werden über ein bewegbares Siebdruckelement 21 und über die Druckerdüsen 19 die jeweils gewünschten Grundkomponenten 20a und 20b auf das Trägerelement 7 bzw. auf zuvor gespritzte weitere Schichten 4, 5 oder 6 aufgebracht. Natürlich werden beim Aufdrucken bereits die später gewünschten Formen der Zuleitung 3 und des Elektrodenkopfes 2 berücksichtigt, wobei als einfachste Variante die Zuleitung 3 als gerades, längliches Element und der Elektrodenkopf 2 als relativ kompaktes Element aufgedruckt wird.

Alternativ wird in Fig. 6 eine Draufsicht auf ein Trägerelement 7 nach dem Bedrucken gezeigt, wobei im oberen Bereich ein relativ langes Zuleitungskabel 3 mit den Schichten 4, 5 und 6 aufgedruckt ist, wobei nach dem Trocknen der Druckbestandteile mittels eines entsprechenden, durch die Schere dargestellten Zuschnitts 22 ein relativ langes Zuleitungskabel geschaffen werden kann. In ähnlicher Art und Weise kann ein spiralförmiger Druck und Zuschnitt - wie im unteren Bereich der Fig. 6 dargestellt - zu einem relativ langen Zuleitungskabel 3 führen. Grundsätzlich ist aber bevorzugt vorgesehen, dass das Zuleitungskabel 3 relativ gerade ist und eine Länge zwischen 30 und 150 cm, vorzugsweise zwischen 50 und 100 cm, aufweist.

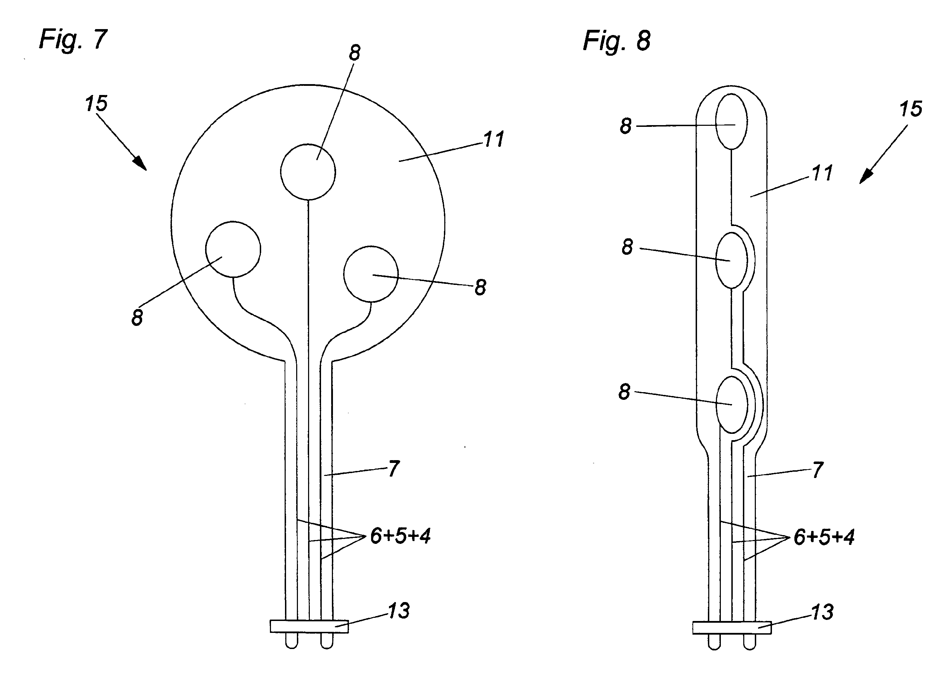

In den Fig. 7 und 8 sind Mehrfachelektroden 15 dargestellt, bei denen auf ein Trägerelement 7 bzw. 11 mehrere voneinander separate Schichten 4, 5 und 6 bzw. 8, 9 und 10 aufgebracht werden.

Als Trägerelement 7 bzw. 11 wird bevorzugt ein nur sehr wenig dehnbarer Kunststoff, wie beispielsweise Polyester (insbesondere PET), verwendet. Es ist aber auch möglich als Trägerelement 7 bzw. 11 eine metallisierte Folie oder sogar eine Polystyrolfolie anzuwenden.

Als Abschirmschicht 4 bzw. 10 können im Druckverfahren Karbontinten oder Metalltinten (beispielsweise Kupfer- oder Silbertinte) verwendet werden. Diese können durchgängig (vollflächig) oder rasterförmig aufgedruckt werden.

Als dielektrisches Element 5 bzw. 9 können im Druckverfahren jegliche Lacke verwendet werden, die nichtleitend und porenlos sind.

Der elektrische Leiter 6 wird bevorzugt als Silberleiter hergestellt, bevorzugt gedruckt. Die Signalabgabe- bzw. -aufnahmeschicht 8 kann in Form einer Silberchloridschicht aufgebracht werden. Auch Zinn und Zinnchlorid können grundsätzlich eingesetzt werden.

Es sei auch noch festgehalten, dass in nahezu allen Druckkomponenten 20a und 20b diverse Zusätze wie Lackelemente, Bindeelemente, Löseelemente usw. vorhanden sind, um einen reibungslosen Druckvorgang zu garantieren und gut aneinander haftende Funktionsschichten erzeugen zu können.

Somit ist der Ersatz (geschirmter) Zuleitungskabel durch eine gedruckte geschirmte Zuleitung möglich, wobei diese idealerweise in einem Produktionsvorgang mit dem Elektrodenkopf (Sensorbereich) zusammen erzeugt (=gedruckt) werden. Dies bringt vor allem Vorteile in Bezug auf die nachfolgende Montage. Insbesondere die sehr aufwändige Montage eines die Schirmung zum Anschluss an das Gerät integrierenden Steckers, die bei geschirmten runden Kabeln erforderlich ist, aber eben auch die hypothetische Montage eines solchen Kabels an eine Elektrode in dem Sinn, dass wiederum ein Abschirmungselement der Elektrode mit der Schirmung des Kabels elektrisch verbunden werden müsste, werden radikal vereinfacht.

## Patentansprüche

1. Medizinische Elektrode (1) mit einem Elektrodenkopf (2) und einer elektrischen Zuleitung (3) zum Elektrodenkopf (2), wobei die Zuleitung (3) eine elektrisch leitende Abschirmschicht (4), ein dielektrisches Element (5) und einen elektrischen Leiter (6) aufweist, wobei die Zuleitung (3) ein längliches Trägerelement (7) aufweist, auf das die elektrisch leitende Abschirmschicht (4) zumindest teilweise aufgedruckt ist, wobei die zumindest teilweise gedruckte Abschirmschicht (4) das dielektrische Element (5) und den elektrischen Leiter (6) quer zur Längsachse (L) der Zuleitung (3) allseitig umgibt, wobei die zumindest teilweise gedruckte Abschirmschicht (4) eine obere (4a) und eine untere (4b) elektrische Abschirmschicht umfasst, und wobei die obere (4a) und die untere (4b) elektrische Abschirmschicht zumindest bereichsweise stoffschlüssig verbunden sind.

2. Medizinische Elektrode nach Anspruch 1, **dadurch gekennzeichnet, dass** das Trägerelement (7) elektrisch nichtleitend ist.

3. Medizinische Elektrode nach Anspruch 1 oder 2, **dadurch gekennzeichnet, dass** das dielektrische Element (5) und/oder der elektrische Leiter (6) gedruckt sind.

4. Medizinische Elektrode nach einem der Ansprüche 1 bis 3, **dadurch gekennzeichnet, dass** die Abschirmschicht (4) zumindest teilweise direkt auf das Trägerelement (7) und das dielektrische Element (5) und der elektrische Leiter (6) indirekt auf das Trägerelement (7) aufgedruckt sind.

5. Medizinische Elektrode nach einem der Ansprüche 1 bis 4, **dadurch gekennzeichnet, dass** die Zuleitung (3) ein oberes (5a) und unteres (5b) dielektrisches Element aufweist, das den elektrischen Leiter (6) quer zur Längsachse (L) der Zuleitung (3) allseitig umgibt.

6. Medizinische Elektrode nach einem der Ansprüche 1 bis 5, **dadurch gekennzeichnet, dass** der Elektrodenkopf (2)
- eine elektrische leitende Signalabgabe- bzw. - aufnahmeschicht (8), die mit dem elektrischen Leiter (6) der Zuleitung (3) verbunden ist,
- ein dielektrisches Element (9), das mit dem dielektrischen Element (5) der Zuleitung (3) verbunden ist und
- eine Abschirmschicht (10), die mit der Abschirmschicht (4) der Zuleitung (3) verbunden ist,
aufweist, wobei zumindest die elektrisch leitende Signalabgabe- bzw. -aufnahmeschicht (8) des Elektrodenkopfs (2), die dielektrische Schicht (9) des Elektrodenkopfs (2) und/oder die Abschirmschicht (4) des Elektrodenkopfs (2) gedruckt ist.

7. Medizinische Elektrode nach Anspruch 6, **dadurch gekennzeichnet, dass** auch der Elektrodenkopf ein elektrisch nichtleitendes Trägerelement (11) aufweist, wobei die Abschirmschicht (10) des Elektrodenkopfs (2) direkt auf dem Trägerelement (11) des Elektrodenkopfs (2) aufgedruckt ist.

8. Medizinische Elektrode nach einem der Ansprüche 1 bis 7, **dadurch gekennzeichnet, dass** eine, vorzugsweise aus Kunststoff bestehende, Schutzschicht (12) die elektrische Zuleitung (3) zumindest teilweise umgibt.

9. Medizinische Elektrode nach einem der Ansprüche 1 bis 8, **dadurch gekennzeichnet, dass** an das elektrodenkopfabgewandte Ende der Zuleitung (3) ein Verbindungsstecker (13) anbringbar bzw. angebracht ist, über den die medizinische Elektrode (1) mit einer medizinischen Auswerte- und/oder Signaleinleitungseinheit verbindbar ist.

10. Medizinische Elektrode nach Anspruch 9, **dadurch gekennzeichnet, dass** die gesamte Abschirmschicht (4), das gesamte dielektrische Element (5) und der gesamte elektrische Leiter (6) zwischen Elektrodenkopf (2) und Verbindungsstecker (13) gedruckt sind.

11. Verfahren zur Herstellung einer medizinischen Elektrode, insbesondere nach einem der Ansprüche 1 bis 10, mit einem Elektrodenkopf (2) und einer elektrischen Zuleitung (3), **gekennzeichnet durch** die Schritte:
- Anordnen eines, vorzugsweise einstückigen, Trägerelements (7, 11) für den Elektrodenkopf (2) und die elektrische Zuleitung (3) in einer Druckvorrichtung (14),
- Drucken einer gemeinsamen, elektrisch leitenden Abschirmschicht (4, 10) auf das Trägerelement (7, 11) im Bereich des Elektrodenkopfs (2) und der Zuleitung (3),
- Drucken eines gemeinsamen, dielektrischen Elements (5, 9) auf die gedruckte, elektrisch leitende Abschirmschicht (4, 10) im Bereich des Elektrodenkopfs (2) und der Zuleitung (3),
- Drucken eines elektrischen Leiters (6) auf das dielektrische Element (5) im Bereich der Zuleitung (3) und einer Signalabgabe- bzw. -aufnahmeschicht (8) im Bereich des Elektrodenkopfs (2), wobei der elektrische Leiter (6) und die Signalabgabe- bzw. -aufnahmeschicht (8) elektrisch leitend verbunden werden,
- Drucken eines oberen, dielektrischen Elements (5a) unter Einschluss des elektrischen Leiters (6) auf das untere, dielektrische Element (5b) zumindest im Bereich der Zuleitung (3), wobei mit dem Drucken das obere (5a) und untere (5b) dielektrische Element stoffschlüssig verbunden werden,
- Drucken einer oberen, dielektrischen Abschirmschicht (4a) unter Einschluss des oberen (5a) und unteren (5b) dielektrischen Elements auf die untere Abschirmschicht (4b) zumindest im Bereich der Zuleitung (3), wobei mit dem Drucken die obere (4a) und untere (4b) Abschirmschicht stoffschlüssig verbunden werden.

12. Verfahren nach Anspruch 11, **dadurch gekennzeichnet, dass** zum Herstellen einer Mehrfach-Elektrode (15) mit zumindest zwei separaten Signafabgabe- bzw. -aufnahmeschichten (8) zumindest zwei separate Abschirmungsschichten (4, 10), zumindest zwei separate dielektrische Elemente (5, 9) und zumindest zwei separate elektrische Leiter (6) auf ein gemeinsames Trägerelement (7, 11) gedruckt werden.

## Claims

1. Medical electrode (1) with an electrode head (2) and an electric feed line (3) to the electrode head (2), wherein the feed line (3) comprises an electrically conductive shielding layer (4), a dielectric element (5) and an electric conductor (6), and wherein the feed line (3) comprises an elongated substrate element (7) onto which the electrically conductive shielding layer (4) is at least partly printed, wherein the at least partly printed shielding layer (4) entirely surrounds the dielectric element (5) and the electric conductor (6) transverse to the longitudinal axis (L) of the feed line (3), wherein the at least partly printed electrically conductive shielding layer (4) comprises an upper (4a) and a lower (4b) electrical shielding ply, and wherein the upper (4a) and the lower (4b) electrical shielding ply are at least partly cohesively joined to one another.

2. Medical electrode according to claim 1, **characterised in that** the substrate element (7) is electrically non-conductive.

3. Medical electrode according to claim 1 or 2, **characterised in that** the dielectric element (5) and/or the electric conductor (6) are printed.

4. Medical electrode according to one of claims 1 to 3, **characterised in that** the shielding layer (4) is printed at least partly directly onto the substrate element (7) and the dielectric element (5) and the electric conductor (6) are printed indirectly onto the substrate element (7).

5. Medical electrode according to one of claims 1 to 4, **characterised in that** the feed line (3) comprises an upper (5a) and a lower (5b) dielectric element, which completely surrounds the electric conductor (6) transverse to the longitudinal axis (L) of the feed line (3).

6. Medical electrode according to one of claims 1 to 5, **characterised in that** the electrode head (2) comprises
- an electrically conductive signal transmitting and receiving layer (8) that is connected to the electric conductor (6) of the feed line (3),
- a dielectric element (9) that is connected to the dielectric element (5) of the feed line (3), and
- a shielding layer (10') that is connected to the shielding layer (4) of the feed line (3),
wherein at least the electrically conductive signal transmitting and receiving layer (8) of the electrode head (2), the dielectric layer (9) of the electrode head (2) and/or the shielding layer (4) of the electrode head (2) are printed.

7. Medical electrode according to claim 6, **characterised in that** also the electrode head comprises an electrically non-conductive substrate element 11, wherein the shielding layer 10 of the electrode head (2) is printed directly onto the substrate element (11) of the electrode head (2).

8. Medical electrode according to one of claims 1 to 7, **characterised in that** a protective layer (12), preferably consisting of plastic material, at least partly surrounds the electric feed line (3).

9. Medical electrode according to one of claims 1 to 8, **characterised in that** a connecting plug (13) can be installed or mounted on the end of the feed line (3) remote from the electrode head, via which the medical electrode (1) can be connected to a medical evaluation and/or signal initiation unit.

10. Medical electrode according to claim 9, **characterised in that** the whole shielding layer (4), the whole dielectric element (5) and the whole electric conductor (6) are printed between the electrode head (2) and connecting plug (13).

11. Method for producing a medical electrode, in particular according to one of claims 1 to 10, with an electrode head (2) and an electric feed line (3), **characterised by** the following steps:
- arranging a preferably one-piece substrate element (7, 11) for the electrode head and the electric feed line (3) in a printing device (14),
- printing a common, electrically conductive shielding layer (4, 10) onto the substrate element (7, 11) in the region of the electrode head (2) and the feed line (3),
- printing a common, dielectric element (5, 9) onto the printed, electrically conductive shielding layer (4, 10) in the region of the electrode head and the feed line (3),
- printing an electric conductor (6) onto the dielectric element (5) in the region of the feed line (3) and printing a signal transmitting and receiving layer (8) in the region of the electrode head (2), wherein the electric conductor (6) and the signal transmitting and receiving layer (8) are electrically connected to one another,
- printing an upper, dielectric element (5a) with inclusion of the electric conductor (6) onto the lower, dielectric element (5b) at least in the region of the feed line (3), wherein the upper (5a) and lower (5b) dielectric elements are cohesively joined to one another by the printing,
- printing an upper, dielectric shielding ply (4a) with the inclusion of the upper (5a) and lower (5b) dielectric element onto the lower shielding ply (4b) at least in the region of the feed line (3), wherein the upper (4a) and lower (4b) shielding plies are cohesively joined to one another by the printing.

12. Method according to claim 11, **characterised in that** for producing a multiple electrode (15) with at least two separate signal transmitting and receiving layers (8) at least two separate shielding layers (4, 10), at least two separate dielectric elements (5, 9) and at least two separate electric conductors (6) are printed onto a common substrate element (7, 11).

## Revendications

1. Electrode médicale (1), avec une tête d'électrode (2) et une ligne d'alimentation (3) électrique de la tête d'électrode (2), la ligne d'alimentation (3) présentant une couche de blindage (4) électriquement conductrice, un élément (5) diélectrique et un conducteur (6) électrique, la ligne d'alimentation (3) présentant un élément porteur (7) oblong sur lequel la couche de blindage (4) électriquement conductrice est imprimée au moins partiellement, la couche de blindage (4) imprimée au moins partiellement entourant de tout côté l'élément (5) diélectrique et le conducteur (6) électrique transversalement par rapport à l'axe longitudinal (L) de la ligne d'alimentation (3), la couche de blindage (4) imprimée au moins partiellement comprenant une couche de blindage électrique supérieure (4a) et une couche de blindage électrique inférieure (4b), et la couche de blindage électrique supérieure (4a) et la couche de blindage électrique inférieure (4b) étant raccordées par combinaison de matière au moins par tronçons.

2. Electrode médicale selon la revendication 1, **caractérisée en ce que** l'élément porteur (7) n'est pas électriquement conducteur.

3. Electrode médicale selon la revendication 1 ou 2, **caractérisée en ce que** l'élément (5) diélectrique et/ou le conducteur (6) électrique sont imprimés.

4. Electrode médicale selon une des revendications 1 à 3, **caractérisée en ce que** la couche de blindage (4) est imprimée au moins partiellement directement sur l'élément porteur (7), et l'élément (5) diélectrique et le conducteur (6) électrique sont imprimés indirectement sur l'élément porteur (7).

5. Electrode médicale selon une des revendications 1 à 4, **caractérisée en ce que** la ligne d'alimentation (3) présente un élément diélectrique supérieur (5a) et un élément diélectrique inférieur (5b) qui entoure de tous côtés le conducteur (6) électrique transversalement à l'axe longitudinal (L) de la ligne d'alimentation (3).

6. Electrode médicale selon une des revendications 1 à 5, **caractérisée en ce que** la tête d'électrode (2) présente
- une couche de délivrance ou respectivement d'enregistrement de signaux (8) électriquement conductrice qui est raccordée au conducteur (6) électrique de la ligne d'alimentation (3),
- élément (9) diélectrique qui est raccordé à l'élément (5) diélectrique de la ligne d'alimentation (3) et
- une couche de blindage (10) qui est raccordée à la couche de blindage (4) de la ligne d'alimentation (3),
au moins la couche de délivrance ou respectivement d'enregistrement de signaux (8) électriquement conductrice de la tête d'électrode (2), la couche diélectrique (9) de la tête d'électrode (2) et/ou la couche de blindage (4) de la tête d'électrode (2) étant imprimées.

7. Electrode médicale selon la revendication 6, **caractérisée en ce que** la tête d'électrode présente également un élément porteur (11) électriquement non conducteur, la couche de blindage (10) de la tête d'électrode (2) étant imprimée directement sur l'élément porteur (11) de la tête d'électrode (2).

8. Electrode médicale selon une des revendications 1 à 7, **caractérisée en ce qu'**une couche de protection (12), de préférence composée de matière plastique, entoure au moins partiellement la ligne d'alimentation (3) électrique.

9. Electrode médicale selon une des revendications 1 à 8, **caractérisée en ce que**, sur l'extrémité de la ligne d'alimentation (3) qui est éloignée de la tête d'électrode, un connecteur (13) peut être mis en place ou est mis en place, par le biais duquel l'électrode médicale (1) peut être raccordée à une unité médicale d'introduction de signaux et/ou d'exploitation.

10. Electrode médicale selon la revendication 9, **caractérisée en ce que** la totalité de la couche de blindage (4), la totalité de l'élément (5) diélectrique et la totalité du conducteur (6) électrique sont imprimées entre la tête d'électrode (2) et le connecteur (13).

11. Procédé de fabrication d'une électrode médicale, en particulier selon une des revendications 1 à 10, avec une tête d'électrode (2) et une ligne d'alimentation (3) électrique, **caractérisé par** les étapes suivantes :
- disposition d'un élément porteur (7, 11), de préférence d'une seule pièce, pour la tête d'électrode (2) et pour la ligne d'alimentation (3) électrique dans un dispositif d'impression (14),
- impression d'une couche de blindage (4, 10) électriquement conductrice commune sur l'élément porteur (7, 11) dans la zone de la tête d'électrode (2) et de la ligne d'alimentation (3),
- impression d'un élément (5, 9) diélectrique commun sur la couche de blindage (4, 10) électriquement conductrice imprimée dans la zone de la tête d'électrode (2) et de la ligne d'alimentation (3),
- impression d'un conducteur (6) électrique sur l'élément (5) diélectrique dans la zone de la ligne d'alimentation (3) et d'une couche de délivrance ou respectivement de captage de signaux (8) dans la zone de la tête d'électrode (2), le conducteur (6) électrique et la couche de délivrance ou respectivement de captage de signaux (8) étant raccordés de façon électriquement conductrice,
- impression d'un élément diélectrique supérieur (5a) avec inclusion du conducteur (6) électrique sur l'élément diélectrique inférieur (5b) au moins dans la zone de la ligne d'alimentation (3), l'élément diélectrique supérieur (5a) et l'élément diélectrique inférieur (5b) étant, avec l'impression, raccordés par combinaison de matière,
- impression d'une couche de blindage diélectrique supérieure (4a) avec inclusion de l'élément diélectrique supérieur (5a) et de l'élément diélectrique inférieur (5b) sur la couche de blindage inférieure (4b) au moins dans la zone de la ligne d'alimentation (3), la couche de blindage supérieure (4a) et la couche de blindage inférieure (4b) étant, avec l'impression, raccordées par combinaison de matière.

12. Procédé selon la revendication 11, **caractérisé en ce que**, pour la fabrication d'une électrode multiple (15) avec au moins deux couches de délivrance ou respectivement d'enregistrement de signaux (8) séparées, au moins deux couches de blindage (4, 10) séparées, au moins deux éléments (5, 9) diélectriques séparés et au moins deux conducteurs (6) électriques séparés sont imprimés sur un élément porteur (7, 11) commun.
